# FASCICULE DE BREVET EUROPEEN

(11) **EP 1 015 741 B1**
(45) Date de publication et mention de la délivrance du brevet: **21.05.2003**
(21) Numéro de dépôt: 99906304.3
(22) Date de dépôt: 25.02.1999
(51) Int. Cl.: F01N 3/28, F01N 3/20

(54) **PROCEDE DE TRAITEMENT DES GAZ D'ECHAPPEMENT D'UN MOTEUR A COMBUSTION INTERNE ET LIGNE D'ECHAPPEMENT ASSOCIEE**
METHODE ZUR ABGASBEHANDLUNG EINER BRENNKRAFTMASCHINE UND ZUGEHÖRIGE AUSPUFFLEITUNG
METHOD FOR TREATING AN INTERNAL COMBUSTION ENGINE EXHAUST GASES AND ASSOCIATED EXHAUST LINE

(30) Priorité: 27.02.1998 FR 9802439
(43) Date de publication de la demande: 05.07.2000
(73) Titulaire: INSTITUT FRANCAIS DU PETROLE, 92506 Rueil Malmaison Cédex (FR)
(72) Inventeur: CASTAGNA, Franck, F-92500 Rueil-Malmaison (FR); MARTIN, Brigitte, F-69230 Saint Genis Laval (FR)
(86) Numéro de dépôt international: FR9900432
(87) Numéro de publication internationale: WO99043934

(56) Documents cités:
- EP-A- 0 563 882
- GB-A- 2 313 796
- PATENT ABSTRACTS OF JAPAN vol. 016, no. 416 (M-1304), 2 septembre 1992 & JP 04 140413 A (YAMAHA MOTOR CO LTD), 14 mai 1992
- PATENT ABSTRACTS OF JAPAN vol. 014, no. 436 (M-1027), 18 septembre 1990 & JP 02 173312 A (MAZDA MOTOR CORP), 4 juillet 1990
- PATENT ABSTRACTS OF JAPAN vol. 017, no. 043 (M-1360), 27 janvier 1993 & JP 04 262016 A (TOYOTA MOTOR CORP), 17 septembre 1992
- PATENT ABSTRACTS OF JAPAN vol. 095, no. 007, 31 août 1995 & JP 07 097916 A (HONDA MOTOR CO LTD), 11 avril 1995

## Description

La présente invention concerne le domaine du traitement des gaz d'échappement issus d'un moteur à combustion interne.

Plus précisément l'invention a trait à la dépollution catalytique de ces gaz d'échappement.

Dans certaines applications (en catalyse trois-voies ou dans la dépollution des moteurs deux-temps), un problème important provient de la forte exothermicité des réactions au niveau des catalyseurs habituellement disposés dans la ligne d'échappement. On appellera dans la suite du texte "catalyseur" ou "monolithe" tout élément comprenant un support mécanique sur lequel est disposé le catalyseur lui-même. L'exothermicité provoque des élévations importantes de la température des monolithes; ces températures sont dangereuses pour la tenue mécanique du support (métal, céramique ou autre). En outre, ces températures élevées sont néfastes à l'activité catalytique du catalyseur déposé sur le support qui va se dégrader après quelques dizaines de milliers de kilomètres parcourus par le véhicule.

Dans d'autres applications (catalyse DéNOx ou catalyse d'oxydation pour moteurs Diesel par exemple), les niveaux de température sont moins élevés mais une moindre efficacité catalytique peut être rencontrée car le catalyseur ne fonctionne pas en permanence dans la plage de température la mieux adaptée au système de dépollution employé.

Afin de résoudre ces problèmes, plusieurs concepts ont déjà été proposés. Un certain nombre d'entre eux divulguent des lignes d'échappement équipées d'un ou plusieurs monolithes dans lesquels un ou plusieurs systèmes de vannage permettent de choisir à chaque instant le ou les monolithes dans lesquels le gaz d'échappement va être dirigé. Le choix des monolithes alimentés en gaz est fonction de différents critères, tels par exemple la température ou le débit des gaz.

Le brevet US 5 377 486 en est un exemple. Dans une telle ligne d'échappement, un premier monolithe dit de "light-off" ou "d'amorçage" est disposé près de la sortie du moteur, un monolithe principal se situant en aval de celui-ci. Le premier monolithe n'est alimenté en gaz d'échappement qu'au démarrage du moteur, tant que les gaz d'échappement sont encore trop froids pour espérer la mise en action du monolithe principal. Situé au plus près du moteur et de taille réduite, ce premier monolithe est cependant efficace rapidement et permet de suppléer en partie l'inactivité du monolithe principal au démarrage. Lorsque les gaz deviennent suffisamment chauds, un vannage piloté permet de ne plus faire passer les gaz dans le premier monolithe, ils vont directement vers le monolithe principal qui s'est mis en action. Il s'agit ainsi de préserver le catalyseur de "light-off".

Le document JP-08 189344 divulgue ainsi un vannage en fonction de la température des gaz en sortie du moteur.

Un autre concept connu, tel que décrit dans le brevet US 3 796 546 par exemple, consiste à faire passer les gaz par des chemins différents, selon leur température. Ainsi, selon ce document, les gaz passent soit successivement à travers deux catalyseurs soit n'en traversent aucun.

Ces concepts ont un coût non négligeable, lié à la nécessité d'utiliser des systèmes de vannage pilotés en fonction de diverses caractéristiques de fonctionnement des véhicules. Ils nécessitent donc la présence supplémentaire de plusieurs capteurs dans la ligne d'échappement ainsi que le développement de stratégies de contrôle.

La problématique exposée ci-dessus, en relation avec la température de la réaction catalytique est abordée, selon l'invention, d'une façon nouvelle et originale.

La présente invention propose en effet de limiter la température au niveau du ou des catalyseurs présents dans la ligne d'échappement en fractionnant le catalyseur et en refroidissant partiellement les gaz d'échappement entre chaque fraction du catalyseur.

Ainsi, la présente invention a pour objet une ligne d'échappement de moteur à combustion interne comprenant une conduite ayant une première extrémité reliée à la sortie du moteur et comprenant plusieurs monolithes ou groupes de monolithes destinés à la conversion catalytique des gaz d'échappement disposés en série dans ladite conduite et séparés les uns des autres par des espaces vides.

Selon l'invention, Ladite ligne comprend en outre un moyen destiné à faire passer de façon permanente une première partie des gaz d'échappement uniquement à travers l'un au moins desdits monolithes ou groupe de monolithes et la seconde partie des gaz d'échappement à travers tous les monolithes, ce qui permet de limiter les pics de température supportés par lesdits monolithes et/ou de les faire fonctionner dans une plage de température déterminée, (à comparer avec JP-A-04 140 413).

Selon l'invention, le premier monolithe ou groupe de monolithes comprend plusieurs monolithes disposés en série et séparés par des espaces vides de monolithe.

Selon un mode de réalisation de l'invention, ladite ligne d'échappement comprend un premier et un deuxième monolithes et un conduit permettant à la première partie du gaz de ne pas passer dans le premier monolithe et d'accéder directement au deuxième monolithe après avoir été remélangée avec la seconde partie du gaz qui, elle, est passée dans le premier monolithe.

Par ailleurs, le deuxième groupe de monolithes comprend plusieurs monolithes disposés en série et séparés par des espaces vides de monolithe.

La ligne d'échappement selon l'invention peut être caractérisée par un premier et un deuxième monolithes disposés dans un carter dans lequel débouche une deuxième extrémité de ladite conduite avec une portion qui dépasse dans ledit carter, des ouvertures étant prévues dans la portion dépassante afin qu'une partie des gaz s'en échappe avant d'atteindre le premier monolithe, et un moyen disposé dans ledit carter permettant de diriger la totalité des gaz à travers le deuxième monolithe.

De façon particulière, le premier monolithe est placé à l'extrémité de la partie dépassante de la conduite.

En outre, le deuxième monolithe peut être disposé sur une paroi de séparation du carter en deux volumes de telle façon qu'il définisse l'unique moyen de passage des gaz d'échappement du premier volume vers le deuxième volume.

L'invention concerne aussi un procédé de traitement des gaz d'échappement issus d'un moteur à combustion interne consistant à les faire passer successivement à travers plusieurs monolithes ou groupe de monolithes destinés à leur conversion catalytique.

Conformément à l'invention, on fait passer en permanence une première partie desdits gaz à travers un premier monolithe ou groupe de monolithes puis à travers un espace sans monolithes puis à travers un deuxième monolithe ou groupe de monolithes et en ce que la seconde partie des gaz ne passe pas à travers le premier monolithe ou groupe de monolithes, afin de limiter les pics de température supportés par lesdits monolithes et/ou de les faire fonctionner dans une plage de température déterminée.

Selon un autre mode de réalisation de l'invention, le procédé utilise un troisième monolithe ou groupe de monolithes disposé en aval du deuxième monolithe, la deuxième partie des gaz est mélangée à la première partie des gaz qui sortent du deuxième monolithe et le mélange est introduit à l'entrée du troisième monolithe ou groupe de monolithes.

D'autres caractéristiques, avantages, détails de l'invention apparaîtront mieux à la lecture de la description qui va suivre, faite à titre illustratif et nullement limitatif en référence aux dessins annexés sur lesquels :
- La figure 1 est un schéma général du principe de l'invention;
- La figure 2 est une coupe schématisée d'un mode de réalisation de l'invention;
- La figure 3 est un schéma d'un autre mode de réalisation de l'invention.

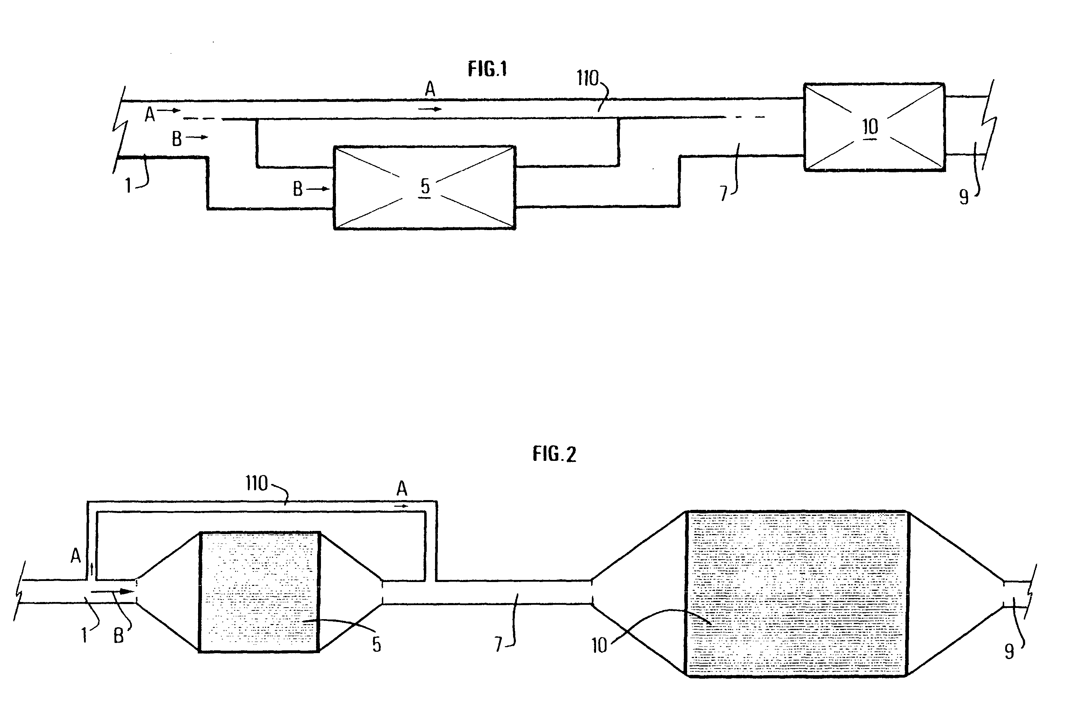

Plus précisément, comme présenté sur la figure 1, ladite ligne d'échappement 1 peut comprendre un premier monolithe 5 ou groupe de monolithes et un deuxième monolithe 10. Un conduit 110 dérive une première partie du gaz sortant du conduit 1 selon les flèches A, qui ne passe pas dans le premier monolithe 5 et est remélangée dans un volume 7 avec la deuxième partie des gaz d'échappement qui, elle, est passée selon les flèches B dans le premier monolithe 5. Ce mélange est effectué en amont du deuxième monolithe 10 de sorte que tout le gaz puisse le traverser avant d'être rejeté dans l'atmosphère. Aucun système de vannage n'est prévu. La proportion de gaz qui ne passe pas dans le premier monolithe 5 est fonction du débit gazeux global du dimensionnement et de l'orientation du conduit de "by-pass" 110.

La figure 2 montre plus précisément une ligne d'échappement ayant un conduit 1 qui sort du moteur. Le conduit 1 est divisé, en amont du premier monolithe 5 de sorte qu'une première partie des gaz d'échappement suit les flèches A, à travers un by-pass 110 du premier monolithe 5. La seconde partie des gaz d'échappement traverse le premier monolithe 5, selon la flèche B. Les deux parties du flux gazeux se rejoignent en amont du deuxième monolithe 10, dans un espace vide 7.

Le mélange entre donc ainsi sur le deuxième monolithe 10 et en ressort par une conduite 9 qui par ailleurs débouche à l'atmosphère.

L'intérêt de l'invention réside en ce que la première partie des gaz, qui ne passe pas à travers le premier monolithe 5, ne contribue à aucune réaction catalytique se produisant dans ce monolithe. On obtient ainsi comme premier effet une limitation de la température dans le monolithe 5. En outre, la première partie des gaz demeure plus froide que la deuxième partie qui, pour sa part, a été réchauffée par son passage dans le premier monolithe 5. Le mélange des deux parties du gaz d'échappement dans le volume 7 va donc permettre d'obtenir un gaz ayant une température moins excessive en entrée du deuxième monolithe 10, et ce de façon permanente. On peut ainsi limiter la température maximale de fonctionnement des monolithes et limiter les risques de dégradation de ceux-ci.

Un tel procédé permet également de demeurer plus longtemps dans les plages de température souhaitées dans les cas où l'on recherche un fonctionnement optimal du catalyseur. Les gaz d'échappement, réchauffés dans le monolithe 5, sont en effet refroidis dans le volume 7 par les gaz qui traversent le conduit 110, avant d'atteindre le deuxième monolithe 10. Ceci peut donc permettre de faire fonctionner le deuxième monolithe 10 à une température voisine de celle du premier monolithe 5 et non pas à une température supérieure qui serait due au réchauffement de l'ensemble des gaz dans le premier monolithe 5 en cas d'absence du conduit 110.

Un autre intérêt du procédé selon l'invention est qu'il ne comprend aucun système de vannage piloté et ne nécessite donc aucun capteur supplémentaire dans la ligne d'échappement. La proportion de gaz passant par le by-pass 110 dépend notamment du débit global des gaz d'échappement, de leur température et de la géométrie de ce conduit 110 qui pourra d'ailleurs être adaptée selon l'application concernée.

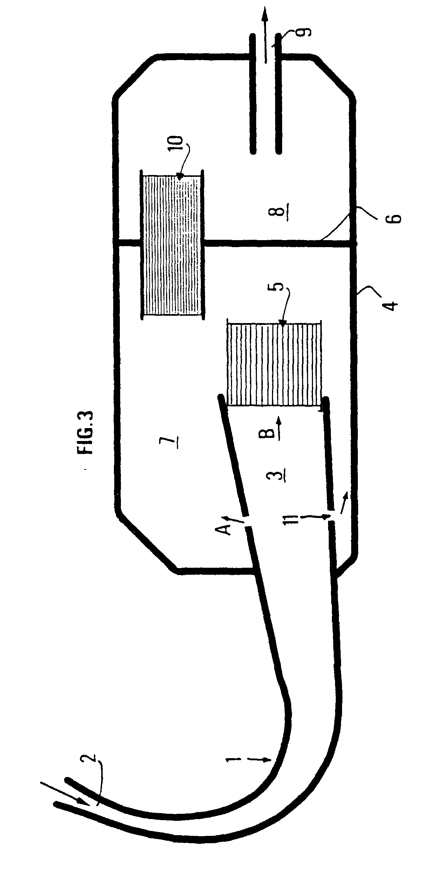

Un deuxième exemple d'application de l'invention est présenté sur la figure 3. Ce type de ligne d'échappement, utilisé par exemple pour les moteurs deux-temps, comprend la conduite 1 ayant une première extrémité 2 reliée à la sortie du moteur (non représenté); à sa deuxième extrémité 3 la conduite débouche, selon ce mode de réalisation de l'invention, dans un carter 4 qui sert habituellement de silencieux. Plus précisément, la conduite 1 dépasse sur une certaine longueur à l'intérieur du carter 4. A l'extrémité 3 est placée un premier monolithe 5 destiné à la conversion catalytique des gaz d'échappement.

En outre, le carter 4 renferme une paroi de séparation 6 qui définit deux volumes; le premier 7 contient l'extrémité 3 de la conduite 1 et le premier monolithe 5.

Le deuxième volume 8 du carter 4 comprend l'ouverture 9 pour la sortie des gaz d'échappement dépollués vers l'atmosphère.

Sur la paroi de séparation 6 est prévu un unique passage qui reçoit le deuxième monolithe 10.

Par ailleurs, selon l'invention, des ouvertures 11 dans la conduite 1 sont prévues à proximité de la deuxième extrémité 3; les ouvertures 11 sont réalisées dans la portion dépassante de la conduite 1 de telle sorte que les gaz qui traversent les ouvertures 11 se retrouvent systématiquement dans le premier volume 7.

Ainsi le cheminement des gaz est le suivant : tous les gaz s'écoulent depuis la première extrémité 2 de la conduite 1 vers la deuxième extrémité 3.

Arrivés au niveau des ouvertures 11, une première partie des gaz s'échappe à travers lesdites ouvertures selon les flèches A, sans subir de réaction catalytique, donc sans subir une augmentation de température. La deuxième partie des gaz est dirigée selon la flèche B à travers le premier monolithe 5, puis entre dans le volume de mélange 7. La température de cette deuxième partie des gaz va s'accroître du fait des réactions catalytiques réalisées dans le premier monolithe 5.

Ainsi, la totalité des gaz se retrouve dans le volume de mélange 7 et en ressort en passant à travers le deuxième monolithe 10 qui est le seul passage vers le deuxième volume 8. La température des gaz en entrée du deuxième monolithe 10 va être limitée par le mélange des deux parties du gaz d'échappement, celle qui est passée dans le monolithe 5 et celle qui est passée par les ouvertures 11. Finalement, la ou les ouvertures 9 constituent la sortie générale des gaz.

Bien entendu, d'autres modes de réalisation permettant à une partie du gaz de contourner en permanence un ou plusieurs monolithes dans la ligne d'échappement peuvent être envisagés par l'homme de métier sans sortir du cadre de la présente invention.

De même, on peut envisager de reproduire ce principe en cascade dans le cas de lignes d'échappement qui contiennent au moins trois monolithes en série séparés par des espaces vides. Dans ce cas la deuxième partie des gaz, qui n'est pas passée par le premier monolithe, peut être mélangée aux gaz sortant du deuxième monolithe à l'entrée du troisième monolithe. A la sortie du premier monolithe les gaz peuvent être divisés pour passer en partie dans le deuxième monolithe et en partie vers la deuxième partie des gaz.

La présente invention trouve son application préférée dans les cas où les températures des gaz d'échappement sont très élevées et peuvent entraîner une détérioration du support du catalyseur ou des performances catalytiques. L'invention peut ainsi limiter les pics de température observés dans les systèmes catalytiques de dépollution des moteurs à allumage commandé et des moteurs deux-temps.

Mais elle peut aussi avantageusement s'appliquer dans les cas où l'on cherche à faire fonctionner le catalyseur dans une fenêtre restreinte de température.

Ainsi dans la dépollution des moteurs à combustion interne, bien que les niveaux de température atteints ne soient pas dangereux pour la tenue des catalyseurs, on observe souvent une conversion optimale des NOx en catalyse DéNOx dans une plage de température comprise entre 200 et 250°C. De même, en catalyse d'oxydation, l'élimination du SO2 qui débute vers 200°C disparaît vers 350°C. L'application de la présente invention dans ces domaines va permettre, lorsque le système de dépollution est placé de façon optimale dans la ligne d'échappement, de limiter au maximum les excursions de températures en dehors de ces plages optimales d'utilisation et d'augmenter l'efficacité globale de la dépollution.

## Revendications

1. Ligne d'échappement de moteur à combustion interne comprenant une conduite (1) ayant une première extrémité (2) reliée à la sortie du moteur et comprenant au moins un premier et un deuxième monolithes ou groupes de monolithes destinés à la conversion catalytique des gaz d'échappement, disposés en série dans ladite conduite et séparés les uns des autres par des espaces vides, comprenant en outre un moyen (110 ; 11) destiné à faire passer de façon permanente une première partie des gaz d'échappement uniquement à travers l'un au moins desdits monolithes ou groupe de monolithes (10) et la seconde partie des gaz d'échappement à travers tous les monolithes (5, 10) afin de limiter les pics de température supportés par lesdits monolithes et/ou de les faire fonctionner dans une plage de température déterminée, **caractérisée en ce que** le premier monolithe ou groupe de monolithes (5) comprend plusieurs monolithes disposés en série et séparés par des espaces vides de monolithes.

2. Ligne d'échappement selon la revendication 1, **caractérisée en ce qu'**elle comprend un conduit (110) permettant à la première partie du gaz de ne pas passer dans le premier groupe de monolithe (5) et d'accéder directement au deuxième monolithe (10) après avoir été mélangée avec la seconde partie du gaz qui, elle, est passée dans le premier groupe de monolithes (5).

3. Ligne d'échappement selon l'une quelconque des revendications 1 ou 2, **caractérisée en ce que** le deuxième groupe de monolithes (10) comprend plusieurs monolithes disposés en série et séparés par des espaces vides de monolithes.

4. Ligne d'échappement selon la revendication 1, **caractérisée en ce que** le premier (5) et le deuxième (10) monolithes sont disposés dans un carter (4) dans lequel débouche une deuxième extrémité (3) de ladite conduite (1) avec une portion qui dépasse dans ledit carter (4), des ouvertures (11) étant prévues dans la portion dépassante afin qu'une partie des gaz s'en échappe avant d'atteindre le premier monolithe (5), et un moyen (6) est disposé dans ledit carter (4) permettant de diriger la totalité des gaz à travers le deuxième monolithe (10).

5. Ligne d'échappement selon la revendication 4, **caractérisée en ce que** le premier monolithe (5) est placé à l'extrémité (3) de la partie dépassante de la conduite (1).

6. Ligne d'échappement selon l'une quelconque des revendications 4 ou 5, **caractérisée en ce que** le deuxième monolithe (10) est disposé sur une paroi de séparation (6) du carter (4) en deux volumes (7, 8) de telle façon qu'il définisse l'unique moyen de passage des gaz d'échappement du premier volume (7) vers le deuxième volume (8).

7. Procédé de traitement des gaz d'échappement issus d'un moteur à combustion interne consistant à les faire passer successivement à travers plusieurs monolithes ou groupe de monolithes destinés à leur conversion catalytique, et consistant en ce qu'on fait passer en permanence une première partie desdits gaz à travers un premier monolithe ou groupe de monolithes puis à travers un espace sans monolithes puis à travers un deuxième monolithe ou groupe de monolithes et en ce que la seconde partie des gaz ne passe pas à travers le premier monolithe ou groupe de monolithes, afin de limiter les pics de température supportés par lesdits monolithes et/ou de les faire fonctionner dans une plage de température déterminée, **caractérisé en ce que** le premier monolithe ou groupe de monolithes comprend plusieurs monolithes disposés en série et séparés par des espaces vides de monolithe.

8. Procédé de traitement selon la revendication 7, **caractérisé en ce qu'**il utilise un troisième monolithe ou groupe de monolithe disposé en aval du deuxième monolithe, **en ce que** la deuxième partie des gaz est mélangée à la première partie des gaz qui sortent du deuxième monolithe et le mélange est introduit à l'entrée du troisième monolithe ou groupe de monolithes.

## Patentansprüche

1. Auspuffleitung für einen Verbrennungsmotor, umfassend eine Leitung (1) mit einem ersten Ende (2) das mit dem Ausgang des Motors verbunden ist und wenigstens einen ersten oder einen zweiten Monolith oder Gruppen von Monolithen umfasst, ausgerichtet auf die katalytische Umwandlung der Auspuffgase, angeordnet in Reihe in der Leitung und voneinander durch Hohlräume getrennt, im übrigen umfassend ein Mittel (110; 11), das ausgerichtet ist, um in permanenter Weise einen ersten Teil der Auspuffgase einzig entlang von einem wenigstens der Monolithe oder Gruppen von Monolithen (10) und den zweiten Teil der Auspuffgase entlang aller Monolithe (5, 10) passieren zu lassen, um die Temperaturspitzen zu begrenzen, die durch die Monolithe unterstützt sind und/oder sie in einem vorgegebenen Temperaturbereich arbeiten zu lassen, **dadurch gekennzeichnet, dass** der erste Monolith oder Gruppe von Monolithen (5) mehrere Monolithe umfasst, die in Reihe angeordnet und durch Hohlräume von Monolithen getrennt sind.

2. Auspuffleitung nach Anspruch 1, **dadurch gekennzeichnet, dass** sie eine Leitung (110) umfasst, welche es dem ersten Teil des Auspuffgases ermöglicht, nicht in die erste Gruppe von Monolithen (5) zu passieren und direkt auf den zweiten Monolith (10) zu treffen, nachdem es mit dem zweiten Teil des Gases gemischt worden ist, welchen es in der ersten Gruppe von Monolithen (5) passiert hat.

3. Auspuffleitung nach einem der Ansprüche 1 oder 2, **dadurch gekennzeichnet, dass** die zweite Gruppe von Monolithen (10) mehrere Monolithe umfasst, die in Reihe angeordnet und durch Hohlräume von Monolithen getrennt sind.

4. Auspuffleitung nach Anspruch 1, **dadurch gekennzeichnet, dass** der erste (5) und zweite (10) Monolith in einem Gehäuse (4) angeordnet sind, in welches ein zweites Ende (3) der Leitung (1) mit einem Teil mündet, welcher das Gehäuse (4) durchschreitet, wobei Öffnungen in dem durchschreitenden Teil vorgesehen sind, damit ein Teil der Gase davon ausweicht, bevor er den ersten Monolith (5) erreicht; und ein Mittel (6) ist in dem Gehäuse (4) angeordnet, welches es ermöglicht, die Gesamtheit der Gase entlang des zweiten Monolithen (10) zu leiten.

5. Auspuffleitung nach Anspruch 4, **dadurch gekennzeichnet, dass** der erste Monolith (5) am Ende (3) des die Leitung durchschreitenden Teils (1) angeordnet ist.

6. Auspuffleitung nach einem der Ansprüche 4 oder 5, **dadurch gekennzeichnet, dass** der zweite Monolith (10) auf einer Wand (6) zum Trennen des Gehäuses (4) in zwei Volumina (7, 8) derart angeordnet ist, dass er einzig ein Durchgangsmittel der Auspuffgase des ersten Volumens (7) zum zweiten Volumen (8) definiert.

7. Behandlungsverfahren der Auspuffgase aus einem Verbrennungsmotor, darin bestehend, aufeinanderfolgend entlang mehrerer Monolithe oder Gruppen von Monolithen, die für deren katalytische Umwandlung vorgesehen sind, sie passieren zu lassen und darin bestehend, dass man permanent einen ersten Teil der Gase entlang eines ersten Monolithen oder Gruppe von Monolithen passieren lässt und dann entlang eines Raumes ohne Monolithen und dann entlang eines zweiten Monolithen oder Gruppe von Monolithen und darin, dass der zweite Teil der Gase nicht entlang des ersten Monolithen oder Gruppe von Monolithen passiert, um die Temperaturspitzen zu begrenzen, die durch die Monolithen unterstützt werden, und/oder sie in einem vorgegebenen Temperaturbereicht arbeiten zu lassen, **dadurch gekennzeichnet, dass** der erste Monolith oder Gruppe von Monolithen mehrere Monolithen umfasst, die in Reihe angeordnet und durch Hohlräume von Monolith getrennt sind.

8. Behandlungsverfahren nach Anspruch 7, **dadurch gekennzeichnet, dass** man einen dritten Monolith oder Gruppe von Monolithen, angeordnet hinter dem zweiten Monolithen, verwendet, dadurch dass der zweite Teil der Gase mit dem ersten Teil der Gase vermischt wird, die den zweiten Monolithen verlassen und die Mischung zum Eingang des dritten Monolithen oder Gruppe von Monolithen eingeführt wird.

## Claims

1. Exhaust system for an internal combustion engine comprising a pipe (1) having a first end (2) connected to the outlet of the engine and comprising at least a first and a second monolith or group of monoliths designed for catalytic conversion of the exhaust gases, disposed in series in said pipe and separated from one another by empty spaces, additionally comprising a means (110; 11) designed to continually pass a first portion of the exhaust gases solely through at least one of said monoliths or groups of monoliths (10) and the second portion of the exhaust gases through all the monoliths (5, 10) in order to limit the temperature peaks borne by said monoliths and/or to make them operate in a given temperature range, ***characterised in that*** the first monolith or group of monoliths (5) comprises a plurality of monoliths disposed in series and separated by empty spaces devoid of monoliths.

2. Exhaust system according to claim 1, ***characterised in that*** it comprises a passage (110) allowing the first portion of the gas not to pass through the first group of monoliths (5) and to pass directly to the second monolith (10) after being mixed with the second portion of the gas which has passed through the first group of monoliths (5).

3. Exhaust system according to any one of claims 1 or 2, ***characterised in that*** the second group of monoliths (10) comprises a plurality of monoliths disposed in series and separated by empty spaces devoid of monoliths.

4. Exhaust system according to claim 1, ***characterised in that*** the first monolith (5) and the second monolith (10) are disposed in a casing (4) into which a second end (3) of said pipe (1) debouches with a portion which projects into said casing (4), openings (11) being provided in the projecting portion so that a portion of the gases escapes before reaching the first monolith (5), and a means (6) is disposed in said casing (4) allowing the entirety of the gases to be directed through the second monolith (10).

5. Exhaust system according to claim 4, ***characterised in that*** the first monolith (5) is placed at the end (3) of the projecting portion of the pipe (1).

6. Exhaust system according to any one of claims 4 or 5, ***characterised in that*** the second monolith (10) is disposed on a wall (6) separating the casing (4) into two spaces (7, 8) such that it defines the sole means for passage of the exhaust gases from the first space (7) to the second space (8).

7. Method for treatment of the exhaust gases coming from an internal combustion engine consisting in passing them successively through a plurality of monoliths or groups of monoliths designed to convert them catalytically, and consisting in continually passing a first portion of said gases through a first monolith or group of monoliths then through a space devoid of monoliths then through a second monolith or group of monoliths, and in not passing the second portion of the gases through the first monolith or group of monoliths in order to limit the temperature peaks borne by said monoliths and/or to operate them within a given temperature range, ***characterised in that*** the first monolith or group of monoliths comprises a plurality of monoliths disposed in series and separated by empty spaces devoid of monoliths.

8. Method for treatment according to claim 7, ***characterised in that*** it uses a third monolith or group of monoliths disposed downstream of the second monolith, **in that** the second portion of the gases is mixed with the first portion of the gases leaving the second monolith and the mixture is introduced into the inlet of the third monolith or group of monoliths.
